# FASCICULE DE BREVET EUROPEEN

(11) **EP 1 757 327 B1**
(45) Date de publication et mention de la délivrance du brevet: **19.08.2009**
(21) Numéro de dépôt: 06291348.8
(22) Date de dépôt: 24.08.2006
(51) Int. Cl.: A61Q 5/10, A61Q 5/04, A61Q 5/06, A61Q 5/08, A61K 8/04, A61K 8/37, A61K 8/22, A61K 8/23, A61K 8/81, A61K 8/87, A61K 8/88, A61K 8/85, A61K 8/41

(54) **Composition oxydante comprenant des composés insolubles, procédés mettant en oeuvre cette composition**
Oxidative Zusammensetzung enthaltend unlösliche Derivate und Verfahren
oxydizing composition comprising unsoluble compounds and processes

(30) Priorité: 25.08.2005 FR 0508754
(43) Date de publication de la demande: 28.02.2007
(60) Demande divisionnaire: 09161939.5
(73) Titulaire: L'Oréal, 75008 Paris (FR)
(72) Inventeur: Kravtchenko, Sylvain, 168 Ji Nan Lu, 200021 Shangai (CN); Dubief, Claude, 78150 Le Chesnay (FR)
(74) Mandataire: Casalonga, Axel

(56) Documents cités:
- EP-A- 0 715 842
- EP-A- 0 823 250
- EP-A- 1 438 951
- WO-A-03/105797
- DE-A1- 2 432 614
- DE-C- 709 932
- US-A- 3 193 464
- US-B1- 6 238 653
- DATABASE WPI Section Ch, Week 200415 Derwent Publications Ltd., London, GB; Class D21, AN 2004-149220 XP002380459 & JP 2004 026703 A (HOYU KK) 29 janvier 2004 (2004-01-29)

## Description

La présente invention a trait à des compositions oxydantes prêtes à l'emploi destinées au traitement des matières kératiniques comprenant un composé insoluble dans l'eau, à des procédé de décoloration ou de décapage, de teinture, d'éclaircissement ou de déformation permanente des fibres kératiniques humaines et en particulier des cheveux et à l'utilisation de ladite composition pour la décoloration, le décapage, la teinture, l'éclaircissement ou la déformation permanente desdites fibres.

Les compositions oxydantes sont utilisées pour éclaircir ou décolorer la fibre kératinique. En effet, la décoloration ou l'éclaircissement des cheveux est obtenu par oxydation des pigments de mélanine, cette oxydation pouvant conduire à l'extrême, à une solubilisation et une élimination totale de ces pigments.

Le but de la décoloration est généralement double. Il peut s'agir soit simplement de donner aux cheveux un aspect plus clair, soit de les préparer à l'application d'un produit de coloration (direct ou d'oxydation), la nuance résultante étant en général plus claire que la nuance naturelle.

Il est connu de décolorer les cheveux à l'aide de pâtes (ou cataplasmes) appliquées directement sur les cheveux et obtenues en mélangeant, au moment de l'emploi, une composition décolorante à base de dérivés peroxydés (agents oxydants) avec de l'eau ou plus habituellement encore avec de l'eau oxygénée. La composition décolorante est, de façon connue, constituée d'un dérivé peroxydé, généralement un persulfate ou un perborate de sodium, de potassium ou d'ammonium et parfois un sel d'acide percarboxylique ou un peroxyde, par exemple de baryum, de strontium, d'urée ou de mélanine. Le plus souvent, ces compositions contiennent en outre, de façon connue, des agents fortement alcalins, tels que des métasilicates, des phosphates ou des carbonates alcalins ou alcalino-terreux (régulateurs de pH). Enfin, elles peuvent par ailleurs éventuellement contenir d'autres additifs ou adjuvants classiques dans le domaine : agents de contrôle du dégagement d'oxygène dans le mélange avec l'eau oxygénée, des épaississants, tels que des dérivés de cellulose (carboxyméthylcellulose par exemple) ou l'amidon et ses dérivés, ou bien encore les gommes de guar, de xanthane et les alginates ; des agents tensio-actifs, en particulier anioniques (alkysulfates notamment) ; des séquestrants ; des parfums. De telles compositions décolorantes sont décrites par exemple, dans « The Science of Hair Car » par C. Zviak, Marcel Decker Inc. 1986, pages 225-226.

Le document US-A-6 238 653 décrit une composition de décoloration des cheveux comprenant de l'ordre de 25% d'huile oxygénée et 2,5 % d'agent oxydant. Le document JP-A-2004 026703 décrit une composition de coloration des cheveux de type émulsion eau-dans-huile comprenant jusqu'à 30 % de paraffine.

Les compositions décolorantes peuvent aussi résulter du mélange, au moment de l'emploi, de la poudre anhydre de réactif péroxygéné avec une composition aqueuse contenant les composés alcalins et une autre composition aqueuse contenant le peroxyde d'hydrogène.

Le processus de décoloration de la fibre dure généralement entre 20 minutes et 1 heure. Ce temps de pose varie selon la forme de la composition oxydante (crème, shampooing, poudre, solution ou émulsion), selon le niveau ou le degré d'éclaircissement souhaité, et selon la concentration des réactifs de la composition oxydante prête à l'emploi, c'est-à-dire la quantité de composés alcalins, de réactifs peroxygénés, le volume d'eau oxygénée ou la proportion de poudres renforçatrices.

On cherche depuis longtemps à accélérer le procédé de décoloration ou d'éclaircissement de la fibre kératinique et à augmenter le degré d'éclaircissement de la fibre, c'est-à-dire à éclaircir plus.

Dans le but de diminuer le temps de pose, il serait tentant d'augmenter la proportion des réactifs au sein de la composition oxydante. Or une concentration plus importante des composants conduirait inévitablement à une altération plus importante de la fibre kératinique, voire à une irritation ou une dermite du cuir chevelu ou l'apparition de picotements.

Les compositions d'oxydation sont également utilisées dans les procédés de déformation permanente, en tant que composition fixante permettant la reconstruction de la fibre dans la forme souhaitée.

Au cours d'un procédé de déformation permanente des cheveux, les cheveux sont enroulés autour de bigoudis, ils sont ensuite imprégnés d'une composition réductrice, dite frisante, que l'on laisse poser entre 5 minutes et 1 heure selon la nature du cheveu et selon le réducteur employé. La forme du cheveu est ensuite fixée à l'aide d'une composition oxydante, qui est appliquée pendant environ 5 minutes, puis laissée poser pendant environ 5 à 30 minutes. Après déroulage des mèches, la composition oxydante est appliquée sur les pointes pendant environ 5 minutes, puis est laissée poser pendant environ 5 minutes. Enfin, la chevelure ainsi traitée est rincée. Ce procédé classique de déformation permanente est long et fastidieux.

Comme pour le procédé de décoloration, il serait souhaitable de mettre au point une composition oxydante plus efficace conduisant à un temps de pose plus court, et diminuant ainsi la durée du procédé de déformation permanente.

Il existe donc un besoin de compositions oxydantes pour la décoloration ou pour la déformation permanente plus efficaces, c'est-à-dire réagissant plus rapidement avec la structure de la fibre, tout en conservant une bonne innocuité vis-à-vis de ladite fibre et du cuir chevelu.

Dans le cadre de la coloration d'oxydation, on procède généralement au mélange au moment de l'emploi d'une composition contenant les colorants d'oxydation habituellement à un pH alcalin, avec une composition oxydante généralement à base d'eau oxygénée formulée à pH acide. Le mélange obtenu est appliqué sur la tête avec des temps de pose pouvant aller jusqu'à une heure. Dans le cas de la coloration indirecte éclaircissante le ou les colorants d'oxydation sont remplacés par au moins un colorant direct.

Il existe un besoin d'accélérer le processus de coloration et/ou de diminuer la concentration en eau oxygénée pour limiter les altérations des fibres kératiniques.

Or, après d'importantes recherches menées sur la question, la demanderesse vient maintenant de découvrir de manière surprenante qu'il est possible d'obtenir des compositions oxydantes prêtes à l'emploi plus efficaces et ayant une bonne innocuité, tout en conservant les proportions des réactifs des compositions oxydantes usuelles. La demanderesse a ainsi mis au point des compositions oxydantes prêtes à l'emploi ne présentant pas les inconvénients précités.

L'invention concerne également les procédés de décoloration, de décapage, de teinture d'oxydation, d'éclaircissement et des procédés de déformation permanente des fibres kératiniques, l'utilisation de ladite composition pour la décoloration, le décapage, la teinture, l'éclaircissement ou la déformation permanente desdites fibres ainsi que des dispositifs de teinture ou « kits » à plusieurs compartiments.

D'autres caractéristiques, aspects, objets et avantages de l'invention figurant dans la description ci-dessous permettront de mieux cerner l'invention.

La présente invention a donc pour objet une composition oxydante prête à l'emploi destinée au traitement des matières kératiniques, telles que les fibres kératiniques humaines, en particulier les cheveux, comprenant dans un milieu cosmétiquement acceptable :
(a) au moins un agent oxydant à une teneur telle que définie à la revendication 1.
(b) au moins 30% en poids d'au moins un composé organique oxygéné non colorant insoluble dans l'eau par rapport au poids total de ladite composition particulier tel que défini à la revendication 1.
(c) au moins 20% en poids d'eau par rapport au poids total de ladite composition.

L'agent oxydant est choisi de préférence parmi le peroxyde d'hydrogène et les composés susceptibles de produire par hydrolyse du peroxyde d'hydrogène tels que le peroxyde d'urée, les bromates de métaux alcalins, les persels tels que les perborates, persulfates, percarbonates, et peroxydes de métaux alcalins ou alcalino-terreux, comme le sodium, le potassium, le magnésium, les peracides ou leurs mélanges ; et les enzymes oxydases parmi lesquelles on peut citer les peroxydases, les oxydo-réductases à 2 électrons telles que les uricases et les oxygénases à 4 électrons comme les laccases, le cas échéant en présence de leur substrat approprié. Le peroxyde d'hydrogène est particulièrement préféré.

La teneur en agent oxydant dans la composition prête à l'emploi est comprise entre 2 et 35 % en poids de la composition prête à l'emploi, de préférence entre 5 et 30 % en poids de la composition prête à l'emploi.

Par composé organique oxygéné, on entend, au sens de la présente invention, tout composé organique comportant au moins un atome d'oxygène dans sa structure moléculaire élémentaire.

Par insoluble dans l'eau selon l'invention, on entend des composés présentant une solubilité dans l'eau à température ambiante (25°C) inférieure à 0,5 % en poids.

Les composés organiques oxygénés de l'invention sont des composés ou non polymériques, choisis parmi les alcools gras et les esters, d'acides ou d'alcools. Concernant les esters, d'acides ou d'alcools gras, les chaînes grasses des acides ou alcools gras comportent de 8 à 40 atomes de carbone et sont éventuellement hydroxylés. Les alcools gras comportent de 8 à 40 atomes de carbone. A titre d'exemple, citons : le mono et le di-stéarate d'éthylène glycol, le monooléate de pentaerythritol, le tristéarate de sorbitan, le dioléate de glycérol, les esters, gras d'éthylène glycol, de propylène glycol, l'alcool stéarylique, l'alcool béhénylique, l'alcool cétylstéarylique.

La teneur en composé insoluble dans l'eau dans la composition prête à l'emploi est supérieure à 30% en poids de la composition oxydante prête à l'emploi, de préférence est comprise entre 30 et 75 % en poids de la composition oxydante prête à l'emploi, et plus particulièrement est comprise entre 30 et 60 % en poids de la composition oxydante prête à l'emploi.

Ainsi lorsque que le ou les composés insolubles dans l'eau selon l'invention sont solides, la composition est sous forme d'une suspension. Lorsque le ou les composés insolubles dans l'eau selon l'invention sont sous forme liquide, par exemple sous forme d'une phase organique insoluble dans la phase aqueuse, la composition est une émulsion, voire une dispersion.

La composition oxydante prête à l'emploi selon l'invention comprend au moins 20% en poids d'eau par rapport au poids total de la composition, de préférence entre 20 et 78 % et encore plus préférentiellement entre 30 et 60 %.

La composition oxydante prête à l'emploi peut en outre comprendre un ou plusieurs solvants organiques.

Le milieu approprié pour les compositions de l'invention est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le glycérol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition oxydante selon l'invention peut contenir en outre des tensioactifs, des polymères épaississants et/ou des polymères amphiphiles pour stabiliser les dispersions, lorsque la composition se trouve sous forme de dispersion.

Il s'agit des matières premières classiquement utilisées. Notons à titre d'exemple :
- les tensioactifs anioniques, non ioniques, cationiques et amphotères possédant une ou plusieurs chaînes alkylées en C₆ à C₂₂, linéaire ou ramifiée.
- les polymères amphiphiles, comme les copolymères à blocs amphiphiles hydrosolubles ou hydrodispersibles, les polymères associatifs porteurs sur leur chaîne principales de groupements alkyles et/ou aryles en C₆ à C₂₂, oxyéthylénés ou non, téléchéliques ou greffés, ces polymères peuvent être de charge cationique, anionique, amphotère ou non ionique,
- les polymères épaississants comme les polymères acryliques réticulés, les polysaccharides naturels ou modifiés.

La composition oxydante prête à l'emploi selon l'invention peuvent également contenir les additifs traditionnellement utilisés dans le domaine en particulier des agents séquestrants tels l'éthylènediaminetétraacétique, le pentasodium pentétate (dénomination CTFA) ou l'acide étidronique ; des agents stabilisants chimiques du peroxyde d'hydrogène tels les sels de stannate et de pyrophosphate de métaux alcalins (comme le sodium, le potassium par exemple), ou le salicylate de sodium ; des parfums ; des agents anti-mousse ; des polymères substantifs cationiques ou amphotères ; des polymères conditionneurs hydrosolubles ou non, ou des chélatants.

Bien entendu, l'homme de l'art veillera à choisir le ou les éventuels composés complémentaires mentionnés ci-avant, afin que les propriétés de la composition oxydante prête à l'emploi ne soient pas, ou substantiellement pas, altérées par la ou les adjonctions envisagées.

Le pH de la composition oxydante prête à l'emploi selon l'invention est avantageusement compris entre 2 et 12, plus particulièrement entre 2,5 et 7, et de préférence entre 3 et 6.

Le pH peut être ajusté classiquement et si nécessaire par ajout d'agents basifiants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

Comme précisé précédemment dans l'introduction, la composition prête à l'emploi selon l'invention peut résulter d'un mélange extemporanée d'une ou plusieurs compositions pulvérulentes anhydres avec au moins une composition aqueuse.

Par décoloration, on entend au sens de la présente invention destruction totale ou partielle des pigments naturels présents dans les fibres kératiniques (en particulier eumélanines et phaéomélanines).

La présente invention a de même pour objet un procédé de décoloration de fibres kératiniques humaines, en particulier des cheveux, consistant à appliquer sur la zone des fibres kératiniques humaines, sèches ou humides, à décolorer, la composition oxydante prête à l'emploi selon l'invention ; à la laisser agir pendant un temps de pose suffisant pour obtenir la décoloration recherchée ; à éliminer la composition par un rinçage à l'eau, suivi d'un lavage avec un shampoing, puis éventuellement d'un séchage.

Le temps de pose varie entre 1 et 60 minutes, plus préférentiellement entre 5 et 40 minutes.

Par décapage, on entend au sens de la présente invention destruction totale ou partielle des pigments ou colorants synthétiques présents sur ou dans les fibres kératiniques résultant d'un procédé de coloration directe ou d'oxydation.

La présente invention porte également sur un procédé de décapage de fibres kératiniques humaines, en particulier des cheveux colorés, consistant à appliquer sur la zone des fibres kératiniques humaines colorés, sèches ou humides, à décaper, la composition de décapage prête à l'emploi selon l'invention ; à la laisser agir pendant un temps de pose suffisant pour obtenir le décapage recherché ; à éliminer la composition par un rinçage à l'eau, suivi d'un lavage avec un shampoing, puis éventuellement d'un séchage.

Le temps de pose varie entre 1 et 60 minutes, plus préférentiellement entre 5 et 40 minutes.

Un autre objet de l'invention est un procédé de teinture d'oxydation des fibres kératiniques humaines et en particulier les cheveux mettant en oeuvre une composition colorante comprenant, dans un support approprié pour la teinture des fibres kératiniques, au moins un précurseur de colorant d'oxydation et une composition oxydante prête à l'emploi telle que définie ci-dessus. Au moment de l'emploi, la composition oxydante prête à l'emploi décrite est appliquée sur les fibres kératiniques avant ou après l'application de la composition colorante; les compositions individuelles sont laissées poser pendant 1 à 60 minutes environ, de préférence 5 à 40 minutes environ, ensuite suit une étape de rinçage, de lavage au shampooing, et éventuellement un rinçage et enfin un séchage. Les compositions individuelles colorante et oxydante décrites peuvent être appliquées dans n'importe quel ordre, avec ou sans rinçage intermédiaire.

La composition colorante mise en oeuvre dans ce procédé de coloration fait intervenir les précurseurs de colorants d'oxydation classiques tels que les bases d'oxydation choisies parmi les phénylènediamines, les bis-phénylalkylènediamines, les para-aminophénols, les ortho-aminophénols, les bases hétérocycliques et leurs sels d'addition ; éventuellement combinés à des coupleurs usuels, comme par exemple les méta-phénylènediamines, les méta-aminphénols, les méta-diphénols, les coupleurs naphtaléniques, les coupleurs hétérocycliques ainsi que leurs sels d'addition.

Un autre objet de l'invention est un procédé de coloration directe éclaircissante des fibres kératiniques humaines et en particulier les cheveux mettant en oeuvre une composition colorante comprenant, dans un support approprié pour la teinture des fibres kératiniques, au moins un colorant direct et une composition oxydante prête à l'emploi telle que définie ci-dessus. Au moment de l'emploi, la composition oxydante prête à l'emploi décrite est appliquée sur les fibres kératiniques avant ou après l'application de la composition colorante; les compositions individuelles sont laissées poser pendant 3 à 40 minutes environ, de préférence 5 à 30 minutes environ, ensuite suit une étape de rinçage, de lavage au shampooing, et éventuellement un rinçage et enfin un séchage. Les compositions individuelles colorante et oxydante décrites peuvent être appliquées dans n'importe quel ordre, avec ou sans rinçage intermédiaire.

Le colorant direct utilisé dans ce procédé de coloration directe éclaircissante est un colorant classique choisi parmi les colorants nitrés de la série benzénique neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

La présente invention a aussi pour objet un procédé de traitement des fibres kératiniques humaines et en particulier les cheveux, en vue d'obtenir une déformation permanente de ces dernières, en particulier sous la forme de cheveux permanentés, ce procédé comprenant les étapes suivantes.

Une composition réductrice est appliquée sur la matière kératinique à traiter, la fibre kératinique étant mise sous tension mécanique avant, pendant ou après ladite application. Celle-ci se fait mèche par mèche ou globalement.

Il convient de manière classique, de laisser reposer pendant quelques minutes, généralement 5 minutes à une heure, la chevelure sur laquelle a été appliquée la composition réductrice. Ainsi, le réducteur a le temps d'agir sur le cheveu. Cette phase d'attente est effectuée de préférence à une température allant de 35°C à 45°C, en protégeant de préférence également les cheveux par un bonnet.

La fibre kératinique imprégnée de la composition réductrice est éventuellement rincée par une composition aqueuse.

La composition oxydante prête à l'emploi de l'invention est appliquée sur la fibre kératinique éventuellement rincée, afin de fixer la nouvelle forme imposée aux cheveux. De nouveau, la chevelure traitée est laissée au repos, pendant 3 à 20 minutes, de préférence entre 5 à 10 minutes.

La fibre kératinique subit, à nouveau, un rinçage, généralement à l'eau.

L'invention a enfin pour objet des dispositifs à plusieurs compartiments, ou « kits », pour la mise en oeuvre des procédés précédemment cités.

Un autre objet de l'invention est un dispositif à 2 compartiments pour la teinture ou pour la déformation permanente ou pour la décoloration des fibres kératiniques en particulier de fibres kératiniques humaines. Un premier compartiment renferme soit une composition de coloration d'oxydation ou directe, soit une composition réductrice, soit une première composition oxydante, comprenant de préférence un sel peroxygéné et un second compartiment renferme la composition oxydante prête à l'emploi définie ci-dessus.

## Revendications

1. Composition oxydante prête à l'emploi destinée au traitement des matières kératiniqnes, telles que les fibres kératiniques humaines, en particulier les cheveux, comprenant dans un milieu cosmétiquement acceptable :
(a) an moins un agent oxydant,
(b) au moins 30% en poids d'au moins un composé organique oxygéné non colorant insoluble dans l'eau c'est-à-dire présentant une solubilité dans l'eau à température ambiante (25°C) inférieure à 0,5 % en poids par rapport au poids total de ladite composition, les composés organiques oxygénés insolubles dans l'eau étant de nature non polymérique, et étant choisis parmi les alcools gras comportant de 8 à 40 atomes de carbone, les esters, d'acides gras ou d'alcool gras, les chaînes grasses des acides ou alcools gras comportant de 8 à 40 atomes de carbone, et
(c) au moins 20% en poids d'eau par rapport au poids total de ladite composition, la teneur en agent oxydant dans la composition prête-à-l'emploi étant comprise entre 2 et 35% en poids de la composition prête à l'emploi.

2. Composition selon la revendication 1, **caractérisée en ce que** l'agent oxydant est choisi parmi le peroxyde d'hydrogène, le peroxyde d'urée, les bromates de métaux alcalins, les perborates, persulfates, percarbonates, et peroxydes de métaux alcalins ou alcalino-terreux, les peracides ou leurs mélanges ; et les enzymes oxydases.

3. Composition selon la revendication 2, **caractérisée en ce que** l'agent oxydant est le peroxyde d'hydrogène.

4. Composition selon l'une quelconque des revendications 1 à 3, **caractérisée en ce que** la teneur en agent oxydant dans la composition prête à l'emploi est comprise entre 5 et 30 % en poids de la composition prête à l'emploi.

5. Composition selon l'une quelconque des revendications précédentes, **caractérisée en ce que** les composés organiques oxygénés insolubles dans l'eau sont choisis parmi le mono et le di-stéarate d'éthylène glycol, le monooléate de pentaerythritol, le tristéarate de sorbitan, le dioléate de glycérol, les esters gras d'éthylène glycol et de propylène glycol, l'alcool stéarylique, l'alcool béhénylique, l'alcool cétylstéarylique.

6. Composition selon l'une quelconque des revendications précédentes, **caractérisée en ce que** la tenseur en composés organiques oxygénés non colorants insolubles dans l'eau dans la composition prête à l'emploi est supérieure à 30% en poids de la composition prête à l'emploi, de préférence est comprise entre 30 et 75 % en poids de la composition prête à l'emploi, et plus particulièrement est comprise entre 30 et 60 % en poids de la composition prête à l'emploi.

7. Composition selon l'une quelconque des revendications précédentes, **caractérisée en ce qu'**elle comprend en outre un ou plusieurs composants parmi les suivants:
- les tensioactifs anioniques, non ioniques, cationiques et amphotère possédant une ou plusieurs chaînes alkylées en C₆ à C₂₂, linéaire ou ramifiée,
- les polymères amphiphiles, comme les copolymères à blocs amphiphiles hydrosolubles ou hydrodispersibles, les polymères associatifs porteurs sur leur chaîne principales de groupements alkyles et/ou aryles en C₆ à C₂₂, oxyéthylénés ou non, téléchéliques ou greffés, de charge cationique, anionique, amphotère on non ionique,
- les polymères épaississants comme les polymères acryliques réticulés, les polysaccharides naturels ou modifiés.

8. Composition selon l'une quelconque des revendications précédentes, **caractérisée en ce qu'**elle comprend les additifs choisis parmi des agents séquestrants tels l'éthylènediaminetétraacétique, le penta sodium pentetate ou l'acide étidronique ; des agents stabilisants chimiques du peroxyde d'hydrogène tels les sels de stannate et de pyrophosphate de métaux alcalins, ou le salicylate de sodium ; des parfums ; des agents anti-mousse ; des polymères substantifs cationiques ou amphotères ; des polymères conditionneurs hydrosolubles on non, ou des chélatants.

9. Composition selon l'une quelconque des revendications précédentes, **caractérisée en ce que** le pH de la composition prête à l'emploi est compris entre 2 et 12, plus particulièrement entre 2,5 et 7, et de préférence entre 3 et 6.

10. Procédé de décoloration de fibres kératiniques humaines, en particulier des cheveux, **caractérisé en ce qu'**il consiste à appliquer sur la zone des fibres kératiniques humaines à décolorer, la composition oxydante prête à l'emploi telle que définie à l'une quelconque des revendications 1 à 9 ; à la laisser agir pendant un temps de pose suffisant pour obtenir la décoloration recherchée ; à éliminer la composition par un rinçage à l'eau, suivi d'un lavage avec un shampoing, puis éventuellement d'un séchage.

11. Procédé de décapage de fibres kératiniques humaines, en particulier des cheveux colorés, **caractérisé en ce qu'**il consiste à appliquer sur la zone des fibres kératiniques humaines colorés à décaper, la composition oxydante prête à l'emploi telle que définie à l'une quelconque des revendications 1 à 9 ; à la laisser agir pendant un temps de pose suffisant pour obtenir le décapage recherché ; à éliminer la composition par un rinçage à l'eau, suivi d'un lavage avec un shampoing, puis éventuellement d'un séchage.

12. Procédé de teinture d'oxydation des fibres kératiniques humaines et en particulier les cheveux, **caractérisé en ce que** l'on applique sur les fibres une composition colorante comprenant, dans un support approprié pour la teinture des fibres kératiniques, au moins un précurseur de colorant d'oxydation et une composition oxydante prête à l'emploi telle que définie à l'une quelconque des revendications 1 à 9, la composition oxydante étant appliquée avant ou après l'application de la composition colorante ; les compositions individuelles sont laissés poser pendant 1 à 60 minutes environ, de préférence 5 à 40 minutes environ, ensuite suit une étape de rinçage, de lavage au shampooing, et suivi éventuellement d'un rinçage et d'un séchage.

13. Procédé de coloration directe éclaircissante des fibres kératiniques humaines et en particulier les cheveux, **caractérisé en ce que** l'on applique sur les fibres une composition colorante comprenant, dans un support approprié pour la teinture des fibres kératiniques, au moins un colorant direct et une composition oxydante prête à l'emploi telle que définie à l'une quelconque des revendications 1 à 9, la composition oxydante étant appliquée sur les fibres kératiniques avant ou après l'application de la composition colorante ; les compositions individuelles sont laissées poser pendant 1 à 60 minutes environ, de préférence 5 à 40 minutes environ, ensuite suit une étape de rinçage, de lavage au shampooing, et éventuellement à nouveau un rinçage et enfin un séchage.

14. Procédé de déformation permanente des fibres kératiniques humaines et en particulier les cheveux, comprenant les étapes suivantes :
- on applique une composition réductrice sur la matière kératinique à traiter, la fibre kératinique étant mise sous tension mécanique avant, pendant ou après ladite application,
- on laisse reposer la chevelure sur laquelle a été appliquée la composition réductrice,
- éventuellement, on rince par une composition aqueuse,
- on applique la composition oxydante prête à l'emploi telle que définie à l'une quelconque des revendications 1 à 9 sur la fibre kératinique,
- on laisse reposer la chevelure sur laquelle a été appliquée la composition oxydante, puis
- on rince par une composition aqueuse.

15. Utilisation de la composition oxydante prête telle que définie à l'une quelconque des revendications 1 à 9 pour la teinture ou pour la réformation permanente ou pour la décoloration ou pour l'éclaircissement ou pour la coloration des fibres kératiniques en particulier de fibres kératiniques humaines.

16. Dispositif à 2 compartiments pour la teinture ou pour la déformation permanente ou pour la décoloration des fibres kératiniques en particulier de fibres kératiniques humaines comprenant un premier compartiment renfermant soit une composition de coloration d'oxydation ou directe, soit une composition réductrice, soit une première composition oxydante contenant de préférence un sel peroxygéné, et un second compartiment renfermant la composition oxydante prête telle que définie à l'une quelconque des revendications 1 à 9.

## Claims

1. Ready-to-use oxidizing composition for treating keratin materials, such as human keratin fibres, in particular the hair, comprising, in a cosmetically acceptable medium:
(a) at least one oxidizing agent,
(b) at least 30% by weight of at least one water-insoluble, that is having a solubility in water at room temperature (25°C) of less than 0.5% by weight, noncolouring oxygenated organic compound relative to the total weight of the said composition, the water-insoluble oxygenated organic compounds being of non-polymeric nature, and being chosen from fatty alcohols containing from 8 to 40 carbon atoms and fatty acid or fatty alcohol esters, the fatty chains of the fatty acids or fatty alcohols containing from 8 to 40 carbon atoms, and
(c) at least 20% by weight of water relative to the total weight of the said composition, the content of oxidizing agent in the ready-to-use composition being between 2% and 35% by weight of the ready-to-use composition.

2. Composition according to Claim 1, **characterized in that** the oxidizing agent is chosen from hydrogen peroxide, urea peroxide, alkali metal bromates, alkali metal or alkaline-earth metal perborates, persulfates, percarbonates or peroxides, and peracids, or mixtures thereof; and oxidase enzymes.

3. Composition according to Claim 2, **characterized in that** the oxidizing agent is hydrogen peroxide.

4. Composition according to any one of Claims 1 to 3, **characterized in that** the content of oxidizing agent in the ready-to-use composition is between 5% and 30% by weight of the ready-to-use composition.

5. Composition according to any one of the preceding claims, **characterized in that** the water-insoluble oxygenated organic compounds are chosen from ethylene glycol monostearate and distearate, pentaerythrityl monooleate, sorbitan tristearate, glyceryl dioleate, fatty esters of ethylene glycol and of propylene glycol, stearyl alcohol, behenyl alcohol and cetylstearyl alcohol.

6. Composition according to any one of the preceding claims, **characterized in that** the content of water-insoluble noncolouring oxygenated organic compounds in the ready-to-use composition is greater than 30% by weight of the ready-to-use composition, preferably between 30% and 75% by weight of the ready-to-use composition and more particularly between 30% and 60% by weight of the ready-to-use composition.

7. Composition according to any one of the preceding claims, **characterized in that** it also comprises one or more components from the following:
- anionic, nonionic, cationic and amphoteric surfactants containing one or more linear or branched C₆-C₂₂ alkyl chains,
- amphiphilic polymers, for instance water-soluble or water-dispersible amphiphilic block copolymers, associative polymers bearing on their main chain oxyethylenated or non-oxyethylenated, telechelic or grafted C₆-C₂₂ alkyl and/or aryl groups, of cationic, anionic, amphoteric or nonionic charge,
- thickening polymers, for instance crosslinked acrylic polymers and natural or modified polysaccharides.

8. Composition according to any one of the preceding claims, **characterized in that** it comprises additives chosen from sequestrants such as ethylenediaminetetraacetic acid, pentasodium pentetate or etidronic acid; chemical hydrogen peroxide stabilizers such as alkali metal stannate and pyrophosphate salts, or sodium salicylate; fragrances; antifoams; cationic or amphoteric substantive polymers; water-soluble or water-insoluble conditioning polymers, or chelating agents.

9. Composition according to any one of the preceding claims, **characterized in that** the pH of the ready-to-use composition is between 2 and 12, more particularly between 2.5 and 7 and preferably between 3 and 6.

10. Process for bleaching human keratin fibres, in particular the hair, **characterized in that** it consists in applying the ready-to-use oxidizing composition as defined in any one of Claims 1 to 9 to the area of human keratin fibres to be bleached; in leaving it to act for a leave-on time that is sufficient to obtain the desired bleaching; in removing the composition by rinsing with water, followed by washing with a shampoo and then optionally by drying.

11. Process for stripping human keratin fibres, in particular dyed hair, **characterized in that** it consists in applying the ready-to-use oxidizing composition as defined in any one of Claims 1 to 9 to the area of dyed human keratin fibres to be stripped; in leaving it to act for a leave-on time that is sufficient to obtain the desired stripping; in removing the composition by rinsing with water, followed by washing with a shampoo and then optionally by drying.

12. Process for the oxidation dyeing of human keratin fibres and in particular the hair, **characterized in that** a dye composition comprising, in a support that is suitable for dyeing keratin fibres, at least one oxidation dye precursor and a ready-to-use oxidizing composition as defined in any one of Claims 1 to 9 is applied to the fibres, the oxidizing composition being applied before or after the application of the dye composition; the individual compositions are left to act for about 1 to 60 minutes and preferably for about 5 to 40 minutes, followed by a step of rinsing, washing with shampoo and then optionally rinsing and drying.

13. Process for the lightening direct dyeing of human keratin fibres and in particular the hair, **characterized in that** a dye composition comprising, in a support that is suitable for dyeing keratin fibres, at least one direct dye and a ready-to-use oxidizing composition as defined in any one of Claims 1 to 9 is applied to the fibres, the oxidizing composition being applied to the keratin fibres before or after the application of the dye composition; the individual compositions are left to act for about 1 to 60 minutes and preferably for about 5 to 40 minutes, followed by a step of rinsing, washing with shampoo and then optionally rinsing again and finally drying.

14. Process for permanently reshaping human keratin fibres and in particular the hair, comprising the following steps:
- a reducing composition is applied to the keratin material to be treated, the keratin fibre being placed under mechanical tension before, during or after the said application,
- the hair on which the reducing composition has been applied is left to stand,
- the hair is optionally rinsed with an aqueous composition,
- the ready-to-use oxidizing composition as defined in any one of Claims 1 to 9 is applied to the keratin fibre,
- the hair on which the oxidizing composition has been applied is left to stand, and then
- the hair is rinsed with an aqueous composition.

15. Use of the ready-to-use oxidizing composition as defined in any one of Claims 1 to 9 for dyeing, permanently reshaping, bleaching, lightening or dyeing keratin fibres and in particular human keratin fibres.

16. Two-compartment device for dyeing, permanently reshaping or bleaching keratin fibres and in particular human keratin fibres, comprising a first compartment containing either a direct or oxidation dye composition, or a reducing composition, or a first oxidizing composition preferably containing a peroxygenated salt, and a second compartment containing the ready-to-use oxidizing composition as defined in any one of Claims 1 to 9.

## Patentansprüche

1. Gebrauchsfertige oxidierende Zusammensetzung, die für die Behandlung von Keratinsubstanzen, wie menschlichen Keratinfasern und insbesondere Haaren vorgesehen ist und in einem kosmetisch akzeptablen Medium enthält:
(a) mindestens ein Oxidationsmittel,
(b) mindestens 30 Gew.-% mindestens einer sauerstoffhaltigen nichtfärbenden organischen Verbindung, die in Wasser nicht löslich ist, d.h. die bei Umgebungstemperatur (25 °C) eine Löslichkeit in Wasser unter 0,5 Gew.-% aufweist, bezogen auf das Gesamtgewicht der Zusammensetzung, wobei die in Wasser nicht löslichen sauerstoffhaltigen organischen Verbindungen von der Art der nichtpolymeren Verbindungen sind und unter den Fettalkoholen mit 8 bis 40 Kohlenstoffatomen und den Estern von Fettsäuren oder Fettalkoholen ausgewählt sind, wobei die Fettketten der Fettsäuren oder Fettalkohole 8 bis 40 Kohlenstoffatome aufweisen, und
(c) mindestens 20 Gew.-% Wasser, bezogen auf das Gesamtgewicht der Zusammensetzung,
wobei der Mengenanteil des Oxidationsmittels in der gebrauchsfertigen Zusammensetzung im Bereich von 2 bis 35 Gew.-% der gebrauchsfertigen Zusammensetzung liegt.

2. Zusammensetzung nach Anspruch 1, **dadurch gekennzeichnet, dass** das Oxidationsmittel unter Wasserstoffperoxid, Harnstoffperoxid, Alkalimetallbromaten, Perboraten, Persulfaten, Percarbonaten und Peroxiden von Alkalimetallen oder Erdalkalimetallen, Persäuren oder deren Gemischen; und Oxidasen ausgewählt ist.

3. Zusammensetzung nach Anspruch 2, **dadurch gekennzeichnet, dass** es sich bei dem Oxidationsmittel um Wasserstoffperoxid handelt.

4. Zusammensetzung nach einem der Ansprüche 1 bis 3, **dadurch gekennzeichnet, dass** der Mengenanteil des Oxidationsmittels in der gebrauchsfertigen Zusammensetzung im Bereich von 5 bis 30 Gew.-% der gebrauchsfertigen Zusammensetzung liegt.

5. Zusammensetzung nach einem der vorhergehenden Ansprüche, **dadurch gekennzeichnet, dass** die sauerstoffhaltigen, in Wasser unlöslichen, organischen Verbindungen unter Ethylenglycolmono- und -distearat, Pentaerythritmonooleat, Sorbitantristearat, Glycerindioleat, Fettsäureestern von Ethylenglycol und Propylenglycol, Stearylalkohol, Behenylalkohol und Cetylstearylalkohol ausgewählt sind.

6. Zusammensetzung nach einem der vorhergehenden Ansprüche, **dadurch gekennzeichnet, dass** der Mengenanteil der nichtfärbenden, sauerstoffhaltigen, in Wasser unlöslichen, organischen Verbindungen in der gebrauchsfertigen Zusammensetzung mehr als 30 Gew.-% der gebrauchsfertigen Zusammensetzung beträgt und vorzugsweise im Bereich von 30 bis 75 Gew.-% der gebrauchsfertigen Zusammensetzung und insbesondere im Bereich von 30 bis 60 Gew.-% der gebrauchsfertigen Zusammensetzung liegt.

7. Zusammensetzung nach einem der vorhergehenden Ansprüche, **dadurch gekennzeichnet, dass** sie ferner eine oder mehrere der folgenden Komponenten enthält:
- anionische, nichtionische, kationische und amphotere grenzflächenaktive Stoffe, die eine oder mehrere geradkettige oder verzweigte Alkylketten mit 6 bis 22 Kohlenstoffatomen aufweisen,
- amphiphile Polymere, wie wasserlösliche oder in Wasser dispergierbare amphiphile Blockcopolymere, assoziative Polymere, die in ihrer Hauptkette Alkyl- und/oder Arylgruppen mit 6 bis 22 Kohlenstoffatomen aufweisen, die ethoxyliert oder nichtethoxyliert, telechelisch oder gepfropft sind, mit kationischer, anionischer, amphoterer oder nichtionischer Ladung,
- verdickende Polymere, wie vernetzte Acrylpolymere, natürliche oder modifizierte Polysaccharide.

8. Zusammensetzung nach einem der vorhergehenden Ansprüche, **dadurch gekennzeichnet, dass** sie Zusatzstoffe enthält, die unter den Maskierungsmitteln, wie Ethylendiamintetraessigsäure, Pentanatriumpentetat oder Etidronsäure; chemischen Stabilisierungsmitteln für Wasserstoffperoxid wie Alkalimetallsalzen von Stannat und Pyrophosphat, oder Natriumsalicylat; Parfums; Schaumverhütungsmitteln; kationischen oder amphoteren substantiven Polymeren; wasserlöslichen oder nichtwasserlöslichen konditionieren Polymeren oder Chelatbildnern ausgewählt sind.

9. Zusammensetzung nach einem der vorhergehenden Ansprüche, **dadurch gekennzeichnet, dass** der pH-Wert der gebrauchsfertigen Zusammensetzung im Bereich von 2 bis 12, insbesondere 2,5 bis 7, und vorzugsweise 3 bis 6 liegt.

10. Verfahren zum Entfärben von menschlichen Keratinfasern und insbesondere Haaren, **dadurch gekennzeichnet, dass** es darin besteht, auf die Bereiche der menschlichen Keratinfasern, die entfärbt werden sollen, die gebrauchsfertige oxidierende Zusammensetzung nach einem der Ansprüche 1 bis 9 aufzutragen; während einer Zeitspanne, die ausreichend ist, um die gewünschte Entfärbung zu erhalten, einwirken zu lassen; die Zusammensetzung durch Spülen mit Wasser gefolgt von einer Wäsche mit einem Haarwaschmittel zu entfernen; und dann gegebenenfalls zu trocknen.

11. Verfahren zum Entfernen der Farbe aus menschlichen Keratinfasern und insbesondere gefärbten Haaren, **dadurch gekennzeichnet, dass** es darin besteht, auf die Bereiche der gefärbten menschlichen Keratinfasern, aus denen die Farbe entfernt werden soll, die gebrauchsfertige oxidierende Zusammensetzung nach einem der Ansprüche 1 bis 9 aufzutragen; während einer Zeitspanne, die ausreichend ist, um die gewünschte Entfärbung zu erhalten, einwirken zu lassen; die Zusammensetzung durch Spülen mit Wasser gefolgt von einer Wäsche mit einem Haarwaschmittel zu entfernen; und dann gegebenenfalls zu trocknen.

12. Verfahren zum oxidativen Färben von menschlichen Keratinfasern und insbesondere Haaren, **dadurch gekennzeichnet, dass** auf die Fasern eine Farbmittelzusammensetzung, die in einem zum Färben von Keratinfasern geeigneten Träger mindestens ein Farbstoffvorprodukt eines Oxidationsfarbstoffes enthält, und eine oxidierende gebrauchsfertigen Zusammensetzung nach einem der Ansprüche 1 bis 9 aufgebracht wird, wobei die oxidierende Zusammensetzung vor oder nach dem Auftragen der Farbmittelzusammensetzung aufgebraucht wird; die einzelnen Zusammensetzungen etwa 1 bis 60 min und vorzugsweise etwa 5 bis 40 min einwirken gelassen werden, worauf ein Spülschritt, das Waschen mit Haarwaschmittel und danach gegebenenfalls ein Spülschritt und Trocknen folgen.

13. Verfahren für die aufhellende Direktfärbung von menschlichen Keratinfasern und insbesondere Haaren, **dadurch gekennzeichnet, dass** auf die Fasern eine Farbmittelzusammensetzung, die in einem zum Färben von Keratinfasern geeigneten Träger mindestens einen Direktfarbstoff enthält, und eine oxidierende gebrauchsfertigen Zusammensetzung nach einem der Ansprüche 1 bis 9 aufgebracht wird, wobei die oxidierende Zusammensetzung vor oder nach dem Auftragen der Farbmittelzusammensetzung aufgebraucht wird; die einzelnen Zusammensetzungen etwa 1 bis 60 min und vorzugsweise etwa 5 bis 40 min einwirken gelassen werden, worauf ein Spülschritt, das Waschen mit Haarwaschmittel und danach gegebenenfalls nochmals ein Spülschritt und Trocknen folgen.

14. Verfahren für die dauerhafte Verformung von menschlichen Keratinfasern und insbesondere Haaren, das die folgenden Schrotte umfasst:
- auf die zu behandelnde Keratinsubstanz wird eine reduzierende Zusammensetzung aufgebracht, wobei die Keratinfaser vor , während oder nach dem Auftragen unter mechanische Spannung gesetzt wird,
- die Haare, auf die die reduzierende Zusammensetzung aufgebracht wurde, werden ruhen gelassen,
- man spült gegebenenfalls mit einer wässrigen Zusammensetzung,
- die oxidierende gebrauchsfertigen Zusammensetzung nach einem der Ansprüche 1 bis 9 wird auf die Keratinfaser aufgebracht,
- die Haare, auf die die oxidierende Zusammensetzung aufgebracht wurde, werden ruhen gelassen, und anschließend
- man spült mit einer wässrigen Zusammensetzung.

15. Verwendung einer oxidierenden gebrauchsfertigen Zusammensetzung nach einem der Ansprüche 1 bis 9 zum Färben oder für die dauerhafte Verformung oder zum Entfärben oder für die Aufhellung oder für die oxidative Färbung von Keratinfasern und insbesondere menschlichen Keratinfasern.

16. Vorrichtung mit zwei Abteilungen zum Färben oder für die dauerhafte Verformung oder zum Entfärben von Keratinfasern und insbesondere menschlichen Keratinfasern, die eine erste Abteilung, die entweder eine Zusammensetzung zum oxidativen Färben oder für die Direktfärbung oder eine reduzierende Zusammensetzung oder eine erste oxidierende Zusammensetzung enthält, die vorzugsweise ein Peroxysalz enthält, und eine zweite Abteilung umfasst, die die gebrauchsfertige oxidierende Zusammensetzung nach einem der Ansprüche 1 bis 9 enthält.
